(19) Europäisches Patentamt
European Patent Office
Office européen des brevets

(11) **EP 4 272 668 A1**

(12) **EUROPEAN PATENT APPLICATION**
published in accordance with Art. 153(4) EPC

(43) Date of publication:
**08.11.2023 Bulletin 2023/45**

(21) Application number: **21912468.2**

(22) Date of filing: **20.01.2021**

(51) International Patent Classification (IPC):
***A61B 18/12*** *(2006.01)*     ***G06F 9/451*** *(2018.01)*

(52) Cooperative Patent Classification (CPC):
**A61B 18/1492;** A61B 2018/00791;
A61B 2018/00875; A61B 2018/00886;
A61B 2018/00898; A61B 2018/00982;
A61B 2034/107; A61B 2034/2051;
A61B 2034/2065; A61B 2034/254; A61B 2090/365

(86) International application number:
**PCT/CN2021/072955**

(87) International publication number:
**WO 2022/141688 (07.07.2022 Gazette 2022/27)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.12.2020   CN 202011641170**

(71) Applicant: **Hangzhou Broncus Medical Co., Ltd.
Hangzhou, Zhejiang 310051 (CN)**

(72) Inventors:
• **XU, Hong
  Hangzhou, Zhejiang 310051 (CN)**
• **CUI, Changjie
  Hangzhou, Zhejiang 310051 (CN)**

(74) Representative: **Petraz, Gilberto Luigi et al
GLP S.r.l.
Viale Europa Unita, 171
33100 Udine (IT)**

(54) **ABLATION OPERATION PROMPTING METHOD, ELECTRONIC DEVICE, AND COMPUTER-READABLE STORAGE MEDIUM**

(57)     Disclosed are an ablation operation prompting method, an electronic device, and a computer-readable storage medium. The method includes: acquiring an image of an ablation site and displaying the image on a screen when an ablation task is triggered; acquiring position data of a currently-being-ablated target ablation point, marking the target ablation point in the image according to the position data; acquiring the elapsed ablation time and the temperature of the target ablation point in real time, and determining the ablation status of the target ablation point according to the elapsed ablation time and the temperature; and generating a schematic real-time dynamic change diagram of the target ablation point according to the ablation status, and displaying the schematic diagram on the screen, to indicate the real-time ablation status change of the target ablation point. In the present invention, the ablation status changes of an ablation site is displayed in real time and visually during the implementation of the ablation operation, to improve the effectiveness of information prompts.

FIG. 3

**Description**

**TECHNICAL FIELD**

The present invention relates to the technical field of data processing, and particularly to an ablation operation prompting method, an electronic device and a computer-readable storage medium.

**BACKGROUND**

Radio-frequency ablation (RFA) is a common treatment method for minimally invasive ablation of tumors. The principle of radio-frequency ablation is to use an alternating high-frequency current with a frequency of less than 30 MHz to cause high-speed oscillations and frictions of ions in tumor tissue, so the radio-frequency energy is converted into heat energy, causing coagulative necrosis of tumor cells.

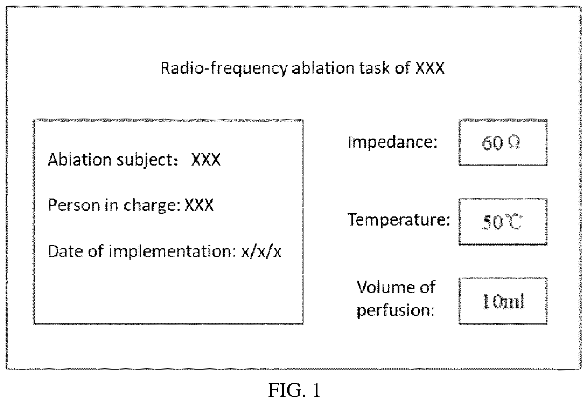

In the prior art, as shown in FIG. 1, when a radio-frequency ablation device performs an ablation operation, generally, the working parameters of the device will be displayed in the form of numbers in an operation interface on a screen, to indicate the status of the current ablation operation. However, this way of prompting is monotonous, contains little information, and tends to be overlooked. Therefore, the prompting effect is poor.

**SUMMARY**

**Technical Problem**

An object of the embodiments of the present invention is to provide an ablation operation prompting method, an electronic device, and a computer-readable storage medium, with which the status changes of an ablation site can be displayed in real time and intuitively during the implementation of the ablation operation, thereby improving the effectiveness and relevance of information prompts.

**Technical solution**

In an aspect, an embodiment of the present application provides an ablation operation prompting method, applicable to a computer terminal. The method includes steps of:

acquiring an image of an ablation site and display the image on a screen, when an ablation task is triggered;
acquiring position data of a currently-being-ablated target ablation point, and marking the target ablation point in the image according to the position data;
acquiring elapsed ablation time and temperature of the target ablation point in real time, and determining the ablation status of the target ablation point according to the elapsed ablation time and the temperature; and
generating a schematic real-time dynamic change diagram of the target ablation point according to the ablation status, and displaying the schematic diagram on the screen, to indicate a real-time ablation status change of the target ablation point.

In an aspect, an embodiment of the present application further provides an ablation operation prompting device, which includes:

an image display module, configured to acquire an image of an ablation site and display the image on a screen, when an ablation task is triggered;
a marking module, configured to acquire position data of a currently-being-ablated target ablation point, and mark the target ablation point in the image according to the position data;
an ablation status determination module, configured to acquire elapsed ablation time and temperature of the target ablation point in real time, and determine the ablation status of the target ablation point according to the elapsed ablation time and the temperature; and
an ablation status prompting module, configured to generate a schematic real-time dynamic change diagram of the target ablation point according to the ablation status, and display the schematic diagram on the screen, to indicate a real-time ablation status change of the target ablation point.

In an aspect, an embodiment of the present application further provides an electronic device, which includes a storage and a processor, wherein

the storage stores an executable program code; and

the processor is coupled to the storage, and configured to call the executable program code stored in the storage, and implement the ablation operation prompting method provided in the above embodiments.

In an aspect, an embodiment of the present application further provides a non-transitory computer-readable storage medium, on which a computer program is stored, wherein when the computer program is executed by the processor, the ablation operation prompting method provided in the above embodiments is implemented.

**Beneficial effects**

According to various embodiments provided in the present application, when an ablation task is triggered, an image of an ablation site is acquired and displayed on a preset prompting interaction interface where the image of the ablation site is marked with a currently-being-ablated target ablation point; according to the elapsed ablation time and the temperature of the target ablation point acquired in real time, a schematic real-time dynamic change diagram of the ablation status of the target ablation point is generated and displayed, so that the status changes of an ablation site can be displayed in real time and intuitively during the implementation of the ablation operation, thereby improving the effectiveness and relevance of information prompts.

**BRIEF DESCRIPTION OF THE DRAWINGS**

In order to describe the technical solutions according to the embodiments of the present invention or in the prior art more clearly, the drawings needed to be used in the embodiments or in the prior art will be described briefly below. Apparently, the drawings in the following description show some embodiments of the present application. Other drawings can be obtained by persons of ordinary skill in the art based on these drawings without creative efforts.

FIG. 1 is a schematic diagram of an existing ablation operation prompting interface;

FIG. 2 shows an application environment of an ablation operation prompting method provided in an embodiment of the present application;

FIG. 3 shows a flow chart of an ablation operation prompting method provided in an embodiment of the present application;

FIG. 4 and FIG. 5 are schematic diagrams showing an image of an ablation site and the real-time dynamic change of the ablation status of a target ablation point in an ablation operation prompting method provided in an embodiment of the present application;

FIG. 6 shows a flow chart of an ablation operation prompting method provided in another embodiment of the present application;

FIG. 7 is a schematic diagram showing an operation track in an ablation operation prompting method provided in an embodiment of the present application;

FIG. 8 is a schematic diagram showing a real-time temperature change curve and a real-time impedance change curve in an ablation operation prompting method provided in an embodiment of the present application;

FIG. 9 is a schematic diagram showing the real-time impedance in an ablation operation prompting method provided in an embodiment of the present application;

FIG. 10 is a schematic diagram showing the real-time impedance change in an ablation operation prompting method provided in an embodiment of the present application;

FIG. 11 is a schematic diagram showing a picture of a screen on which all the schematic views are displayed in an ablation operation prompting method provided in an embodiment of the present application;

FIG. 12 is a schematic structural diagram of an ablation operation prompting device provided in an embodiment of the present application; and

FIG. 13 is a schematic diagram showing a hardware structure of an electronic device provided in an embodiment of the present application.

**DESCRIPTION OF THE EMBODIMENTS**

In order to make the objects, technical solutions and advantages of the embodiments of the present invention clearer, the technical solutions according to the embodiments of the present invention will be clearly and completely described with reference to drawings in the embodiments of the present invention. Apparently, the embodiments described are merely some embodiments, but not all of the embodiments of the present application. All other embodiments obtained by ordinary persons skilled in the art based on the embodiments of the present invention without creative efforts shall fall within the protection scope of the present invention.

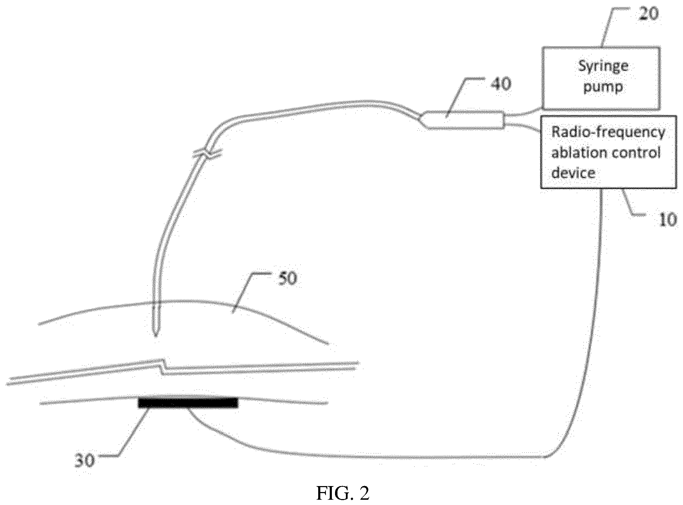

Fig. 2 is a schematic view showing an application scenario of an ablation operation prompting method provided in an embodiment of the present application. The ablation operation prompting method can be implemented by a radio-frequency ablation control device 10 shown in FIG. 2, or implemented by other computer equipment that has established a data connection with the radio-frequency ablation control device 10.

As shown in FIG. 2, the radio-frequency ablation control device 10 is connected to a syringe pump 20, a neutral electrode 30 and a radio-frequency ablation catheter 40. The radio-frequency ablation control device 10 is provided with a built-in display screen (not shown).

Specifically, before an ablation task is implemented, an energy emitting end of the radio-frequency ablation catheter 40 for generating and outputting radio-frequency energy and an extension tube (not shown) of the syringe pump 20 are inserted into the body of an ablation object 50 (such as an emphysema patient), reaching an ablation site. Then, the neutral electrode 30 is brought into contact with the skin surface of the ablation object 50. A radio-frequency current flows through the radio-frequency ablation catheter 40, the tissue of the ablation object and the neutral electrode 30, to form a circuit.

When an ablation task is triggered, the radio-frequency ablation catheter 40 is controlled by the radio-frequency ablation control device 10 to outputting radio-frequency energy to the ablation site by discharging to implement an ablation operation on the ablation site. Meanwhile the syringe pump 20 performs a perfusion operation on the ablation object through the extension tube, wherein physiological saline is infused into the ablation site, to adjust the impedance and temperature of the ablation site.

At the same time, the radio-frequency ablation control device 10 acquires a locally pre-stored image of the ablation site and displays the image on the display screen.

Moreover, the radio-frequency ablation control device 10 also acquires position data of a currently-being-ablated target ablation point by for example, a camera (not shown) installed near the energy emitting end of the radio-frequency ablation catheter, and marks the target ablation point in the image according to the position data.

Further, the radio-frequency ablation control device 10 also acquires the elapsed ablation time and the temperature of the target ablation point in real time by a built-in timer and a temperature sensor (not shown) installed near the energy emitting end, determines the ablation status of the target ablation point according to the elapsed ablation time and the temperature, and then generate a schematic real-time dynamic change diagram of the target ablation point according to the ablation status, and display the schematic diagram on the screen, to indicate to a user the real-time ablation status change of the target ablation point.

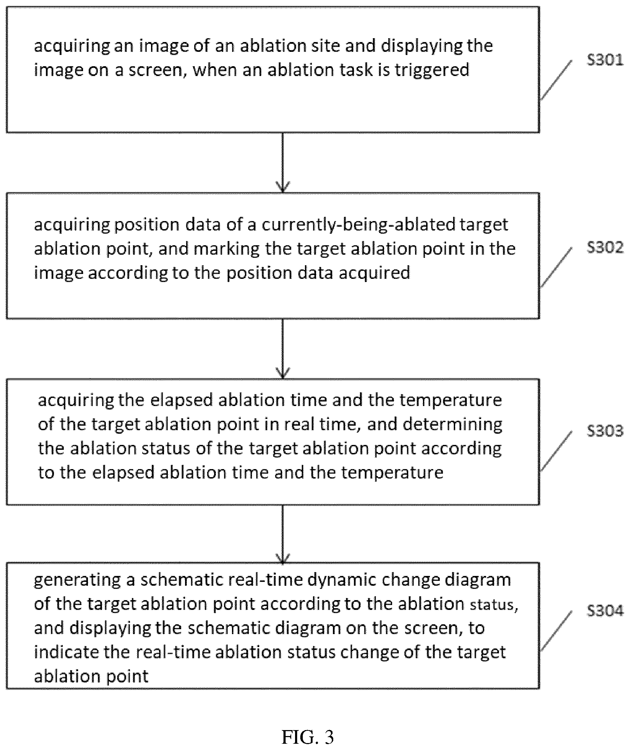

Fig. 3 shows a flow chart of an ablation operation prompting method provided in an embodiment of the present application. The method can be implemented by a radio-frequency ablation control device 10 shown in FIG. 2, or implemented by other computer terminals connected thereto. For ease of description, in the following embodiments, the radio-frequency ablation control device 10 is used as an implementation body. As shown in FIG. 3, the method includes specifically:

Step S301: acquiring an image of an ablation site and displaying the image on a screen, when an ablation task is triggered.

Specifically, before Step S301 is implemented, besides the radio-frequency ablation control device, a main control device of an image acquisition system can also acquire a holographic image of an ablation site in each ablation object intended to receive an ablation operation by an ultrasound medical imaging device or an endoscopic system in advance, and store the acquired holographic image in an image database. When an ablation task is triggered, identification information of a target ablation object corresponding to the ablation task is acquired, and then the image of the ablation site in the target ablation object is acquired according to the identification information by querying the image database, and displayed on a screen.

The screen may be a display screen built in the radio-frequency ablation control device, or an external display screen that has established a data connection with the radio-frequency ablation control device. The holographic image can be an original image taken, or a two-dimensional or three-dimensional static or dynamic image converted from the original image by calling PROE, AUTOCAD, Adobe Photoshop, Python Matplotlib, or other image processing programs.

It can be understood that in addition to PROE, AUTOCAD, Adobe Photoshop, and Python Matplotlib, there are still many other applications currently used for image processing. The program used can be determined according to actual needs, and is not particularly limited in the present application.

Optionally, the preset prompting interaction interface can be used as a carrier of the image and various schematic diagrams involved in the various embodiments of the present application, to facilitate the layout design and the management and positioning of each schematic diagram. For example, the image is displayed in a first preset area of the preset prompting interaction interface.

The preset prompting interaction interface is a graphical user interface (GUI). It can be understood that an application of the prompting interaction interface is preset in the radio-frequency ablation control device. Before the ablation operation is implemented, the application is automatically called, to display the prompting interaction interface on the screen. The preset prompting interaction interface includes multiple areas, respectively used to display different prompt information. The processing logic of the application can automatically divide areas, based on the number of information that needs to be displayed.

Specifically, the acquired image of the ablation site is stored in a first storage location corresponding to a first preset area of the prompting interaction interface. The application automatically refreshes the prompting interaction interface according to a preset period, and adds the image to the first preset area for display if the image is read from the first storage location when implement a refresh operation. Alternatively the acquired image of the ablation site is displayed in the first preset area of the preset prompting interaction interface in the form of overlapped images.

Step S302: acquiring position data of a currently-being-ablated target ablation point, and marking the target ablation point in the image according to the position data.

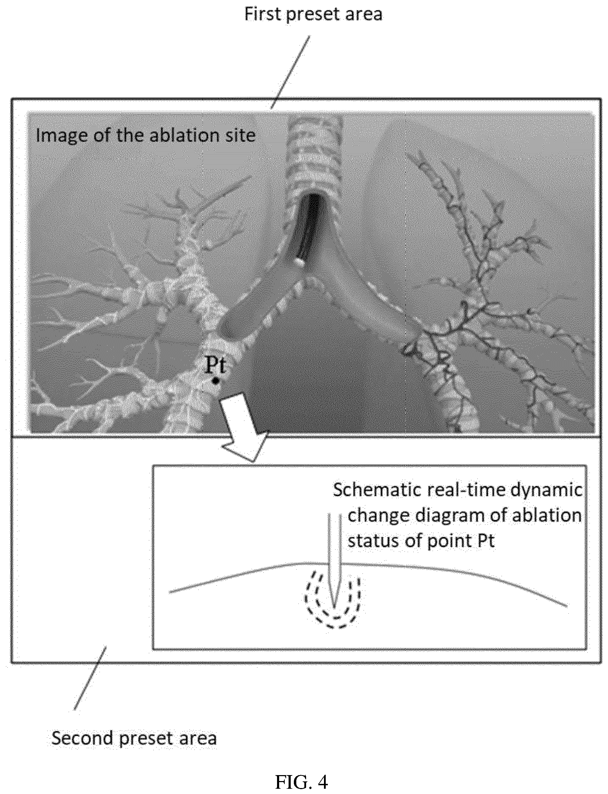

Specifically, position coordinate of the currently-being-ablated target ablation point in the image of the ablation site is acquired according to any one of the following three preset positioning methods. Then, the target ablation point is marked in the image of the ablation site according to the position data and a preset marking logic, wherein the marking logic is a general designation of the preset various operations required for marking as shown in FIG. 4, according to the position coordinate, a circular icon Pt is drawn at a corresponding position in the image and used as a mark.

The first preset positioning method is to obtain the position data of the target ablation point through an endoscope. Specifically, a picture of a current ablation operation captured by an endoscope is acquired and compared with the image, to obtain the position data of the target ablation point in the image.

It can be understood that a camera is provided at the tip of the endoscope; and when an ablation operation is implemented, the camera is inserted together with an ablation catheter into the body of an ablation object, approaching the ablation site, to capture a picture of the ablation site in real time. The camera sends the captured pictures back to the radio-frequency ablation control device while the pictures are captured. The feature points in an area in the ablation site where the ablation operation is being performed in the picture returned by the endoscope are extracted and matched with the feature points in the image displayed in Step S301; and position data corresponding to a feature point with the highest matching degree is determined as the position data of the target ablation point. The position data is a position coordinate, and the coordinate system of the position coordinate is a two-dimensional or three-dimensional coordinate system established by using a centroid of the ablation site in the displayed image or an end point of the image as the origin.

The second preset positioning method is to obtain the position data of the target ablation point through an ultrasound medical imaging device. Specifically, an ultrasound image of the target ablation point is obtained by using an ultrasound medical imaging device; and according to the ultrasound image, the position data of the target ablation point in the image is obtained.

The working principle of the ultrasound medical imaging device is to irradiate the human body with ultrasonic waves, and obtain a visible image of the nature and structure of human tissues, by receiving and processing echoes carrying feature information of nature or structures of human tissues, such as a section shape of the ablation site. At present, many kinds of ultrasound medical imaging devices are available, and the ultrasound medical imaging device used is not particularly limited in the present application. image recognition of the ultrasound image is performed, to determine the position data of the target ablation point in the ultrasound image. Then, the position data of the target ablation point in the displayed image is determined according to the corresponding relationship between each ablation site in the ultrasound image and each ablation site in the image displayed in Step S301.

The third preset positioning method is to obtain the position data of the target ablation point by electromagnetic navigation technology. Specifically, the actual position data of the target ablation point is obtained by for example electromagnetic navigation bronchoscopy (ENB), and then the position data of the target ablation point in the displayed image is determined according to the corresponding relationship between the real ablation site and each ablation site in the image displayed in Step S301.

Step S303: acquiring the elapsed ablation time and the temperature of the target ablation point in real time, and determining the ablation status of the target ablation point according to the elapsed ablation time and the temperature.

Specifically, in an ablation task, at least one ablation site needs to be ablated, each ablation site includes multiple ablation points, and for each ablation point, a corresponding ablation operation needs to be performed. A timer is preset in the radio-frequency ablation control device; and whenever an ablation operation of an ablation point is started, the timer records the elapsed ablation time of the ablation point. When the position of the ablation operation changes, the current timing is ended, and a new round of timing is restarted.

Moreover, during the timing, the temperature near the target ablation point is also obtained by a temperature sensor in real time. Then, according to the elapsed implementation time of the ablation operation (that is, the elapsed ablation time) and the temperature, the ablation status of the target ablation point, for example, the shape and size of the tissue ablated, is determined.

For the same ablation point, timing separately according to the temperature can be performed, that is, the durations at different temperatures are statistically counted. The shape and size of the tissue ablated can be determined according to the length, width and height of the ablated area starting from the target ablation point (hereinafter collectively referred to as the ablated length, width and height of the target ablation point). The ablated length, width and height of the target ablation point are calculated according to Formulas 1-3 below:

Formula 1: (time of a temperature continuously reaching a preset temperature/preset unit of ablation time) * preset ablation length = ablated length;

Formula 2: (time of a temperature continuously reaching a preset temperature/preset unit of ablation time) * preset ablation width = ablated width;

Formula 3: (time of a temperature continuously reaching the preset temperature/preset unit of ablation time) * preset ablation height = ablated height.

The preset temperature and the preset unit ablation time are respectively the critical temperature and the unit critical time allowing the ablation site to undergo qualitative changes (i.e., achieve the expected ablation effect). The preset ablation length, preset ablation height and preset ablation width are the length, width and height of the ablated area increased with the elapse of a preset unit of ablation time after the ablation site reaches the preset temperature. The preset temperature, preset unit of ablation time, and preset ablation length, width and height can be set according to the user's custom operation.

It is experimentally confirmed that after the temperature of the ablation site reaches the critical temperature, the increase in the length, width, and height of the ablated area is generally constant in every time interval. Therefore, according to the above Formulas 1 to 3, the ablated length, width and height of the target ablation point can be obtained. Then, according to the obtained ablated length, width and height and the coordinate of the target ablation point, the shape, size, and boundary coordinates of the ablated area around the target ablation point are obtained.

Step S304: generating a schematic real-time dynamic change diagram of the target ablation point according to the ablation status, and displaying the schematic diagram on the screen, to indicate the real-time ablation status change of the target ablation point.

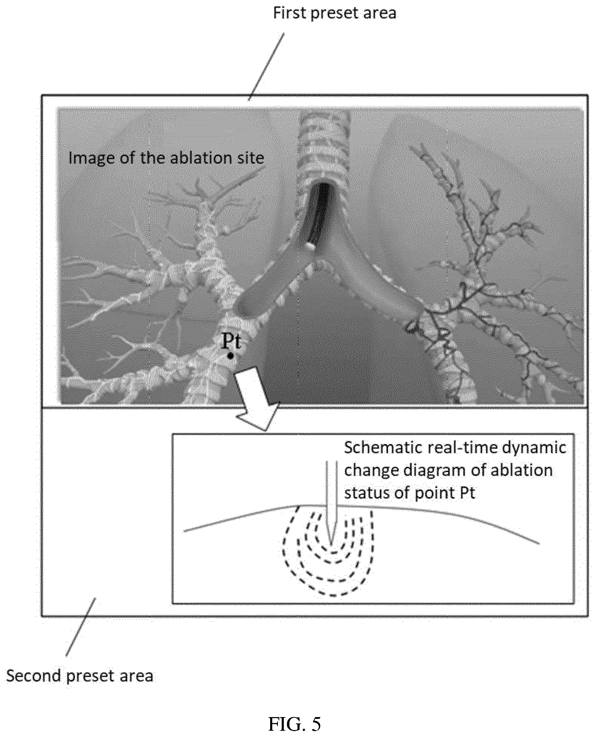

Specifically, by calling the above image processing program, and according to the coordinate of the target ablation point, the shape and size of the ablated area around the target ablation point, and the boundary coordinates of the ablated area, a schematic real-time dynamic change diagram of the ablation status of the target ablation point as shown in FIG. 4 and FIG. 5 is generated. The schematic real-time dynamic change diagram contains visualizations of the process of continuously expanding the boundary of the ablated area. Each circle of dotted lines in the schematic real-time dynamic change diagrams of the ablation status of the target ablation point as shown in FIG. 4 and FIG. 5 indicates the boundary of each expansion of the ablated area. Further, the boundary of the outermost circle can also be displayed in a flashing manner, to make the schematic diagram more indicative.

Optionally, the schematic real-time dynamic change diagram is displayed in a second preset area of the preset prompting interaction interface. The second preset area may be set within an area embraced by the first preset area. Alternatively, it may also be set near the first preset area.

Further, the positional relationship between the first preset area and the second preset area may also be determined according to the number of contents that needs to be displayed in the prompting interaction interface. For example, when there are more contents to be displayed, the generated schematic real-time dynamic change diagram is displayed by overlapping in the vicinity of the target ablation point in the image displayed in the first preset area, to save the space occupied. When there are less content to be displayed, the schematic real-time dynamic change diagram is displayed in an area next to the first preset area, thereby improving the flexibility of information display.

In the embodiments of this application, when an ablation task is triggered, an image of an ablation site is acquired and displayed on a preset prompting interaction interface where the image of the ablation site is marked with a currently-being-ablated target ablation point; according to the elapsed ablation time and the temperature of the target ablation point acquired in real time, a schematic real-time dynamic change diagram of the ablation status of the target ablation point is generated and displayed, so that the status changes of an ablation site can be displayed in real time and intuitively during the implementation of the ablation operation, thereby improving the effectiveness and relevance of information prompts.

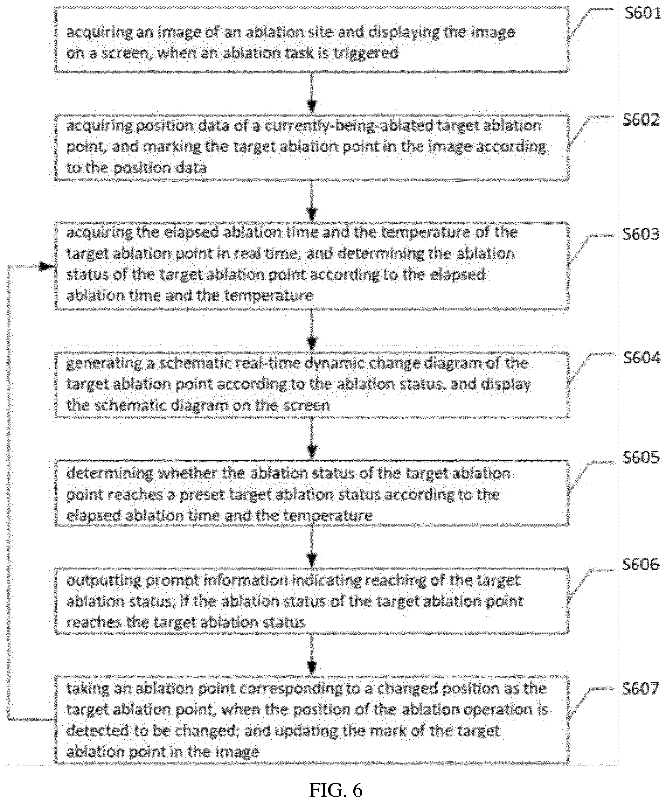

FIG. 6 shows a flow chart of an ablation operation prompting method provided in another embodiment of the present application. The method can be implemented by a radio-frequency ablation control device 10 shown in FIG. 2, or implemented by other computer terminals connected thereto. For ease of description, in the following embodiments, the radio-frequency ablation control device 10 is used as an implementation body. As shown in FIG. 6, the method includes specifically:

Step S601: acquiring an image of an ablation site and displaying the image on a screen, when an ablation task is triggered.

Step S602: acquiring position data of a currently-being-ablated target ablation point, and marking the target ablation point in the image according to the position data.

Step S603: acquiring the elapsed ablation time and the temperature of the target ablation point in real time, and determining the ablation status of the target ablation point according to the elapsed ablation time and the temperature.

Step S604: generating a schematic real-time dynamic change diagram of the target ablation point according to the ablation status, and display the schematic diagram on the screen, to indicate the real-time ablation status change of the target ablation point.

Steps S601 to S604 are the same as Steps S301 to S304 in the embodiment shown in FIG. 3, and details may be made reference to the relevant description in the embodiment shown in FIG. 3, and will not be repeated here.

Step S605: determining whether the ablation status of the target ablation point reaches a preset target ablation status according to the elapsed ablation time and the temperature.

Step S606: outputting prompt information indicating reaching of the target ablation status, if the ablation status of the target ablation point reaches the target ablation status.

Specifically, according to the elapsed ablation time and the temperature of the target ablation point, the ablated length, width and height of the target ablation point are determined, and whether the ablated length, width and height of the target ablation point reach corresponding preset standard values respectively are determined. If they reach the corresponding preset standard values respectively, the ablation status of the target ablation point is determined to reach the preset target ablation status, and prompt information indicating reaching of the target ablation status is outputted. The prompt information indicating reaching of the target ablation status can be output in at least one form of a prompt text, a prompt graphic, a prompt sound, and a prompt light, etc.

Step S607: taking an ablation point corresponding to a changed position as the target ablation point, when the position of the ablation operation is detected to be changed; updating the mark of the target ablation point in the image and returning to Step S603, until the ablation operation is ended.

Specifically, the method described in Step S602 can be used, wherein the position data of the ablation operation is acquired in real time, and whether the position of the ablation operation has changed is detected according to the position data. If the position of the ablation operation is detected to be changed, it means that the ablation point has changed. Therefore, an ablation point corresponding to a changed position is used as a new target ablation point, and a changed position coordinate is used as the position data of the new target ablation point. Then according to the position data, the mark of the target ablation point is updated in the image displayed on the screen. For example, the mark of the previous target ablation point is hidden or deleted, and the mark of the new target ablation point is added to the image at the same time. The method of adding the mark of the new target ablation point is the same as that for the previous target ablation point and will not be repeated here.

Moreover, the process is returned to Step S603: acquiring the elapsed ablation time and the temperature of the target ablation point in real time, and determining the ablation status of the target ablation point according to the elapsed ablation time and the temperature, until the ablation operation is ended. The ablation operation can be automatically ended by the radio-frequency ablation control device when an abnormal event or other preset events are detected, or ended according to the user's operation.

Optionally, in another embodiment of the present application, after Step S601 of acquiring an image of an ablation site and displaying the image on a screen when an ablation task is triggered. the method further includes:

acquiring a first drawing parameter of a target operation track of the ablation operation, and drawing the target operation track at a corresponding position of the image according to the first drawing parameter;

acquiring the position change data of the ablation point in real time, determining a second drawing parameter of the real-time operation track of the ablation operation according to the position change data, and drawing the real-time operation track at a corresponding position of the image according to the second drawing parameter; and

analyzing in real time whether the amplitude of the real-time operation track deviating from the target operation track is greater than a preset amplitude, and outputting prompt information for track deviation warning when the real-time operation track deviates from the target operation track by an amplitude greater than the preset amplitude.

Specifically, the first drawing parameter of the target operation track of each ablation operation to be performed is stored in the radio-frequency ablation control device locally, or in a database server that has established a data connection with the radio-frequency ablation control device. The radio-frequency ablation control device can query the first drawing parameter of the target operation track of the corresponding ablation operation locally or from the database server according to the identification information of the triggered ablation task.

It can be understood that the ablation site is composed of multiple ablation points, the ablation operation needs to be performed on each ablation point one by one. The target operation track is a preset preferred route to be taken when the ablation operation is performed for each ablation point, and used to assist the user in the ablation operation, to achieve a better ablation effect.

Optionally, the first drawing parameter can be generated according to a route parameter input by the user, or calculated according to the preset ablation target and the shape and size of the ablation site.

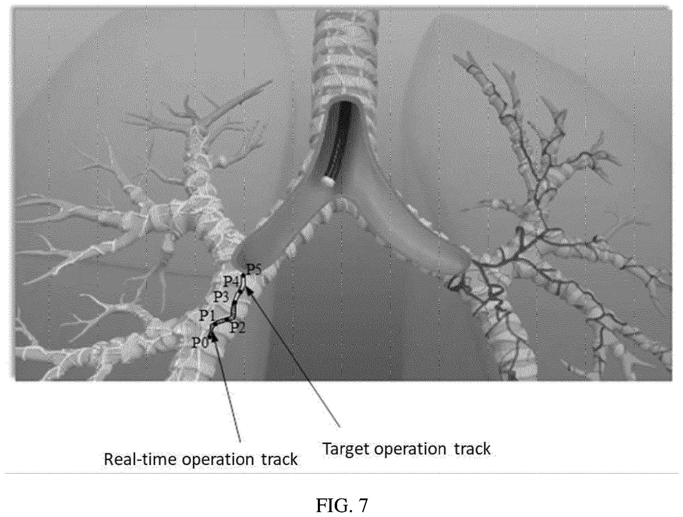

The first drawing parameter may specifically include, but is not limited to, position coordinates and drawing order of various ablation points (such as points P0 to P5 in FIG. 7) in the target operation track, wherein P0 is the starting point, and P5 is the end point), and the line characteristics of the target operation track. The line characteristics of the target operation track can include, but are not limited to, for example: line type (such as: dashed line, or solid line), shadow, thickness, and color, etc.

Further, the method described in Step S302 can be used, wherein the position data of the ablation operation is acquired in real time. Then, the position change data of the ablation operation is obtained according to the position data obtained in real time, wherein the position change data includes: the initial position coordinate of the ablation operation and the position coordinates after each position change. Whenever a position change of the ablation operation is detected, the changed position is marked as an ablation point and the position coordinate of the ablation point is recorded. Then, the second drawing parameter is determined according to the position coordinate of the marked ablation point and the preset drawing logic, and the real-time operation track is drawn according to the determined second drawing parameter (as shown in FIG. 7), to present a display effect of the real-time operation track dynamically extending over time.

The second drawing parameter can include, but is not limited to: position coordinates and drawing order of various ablation points (i.e. various marked ablation points) in the real-time operation track, and the line characteristics of the real-time operation track. The line characteristics of the real-time operation track can include, but are not limited to, for example: line type (such as: dashed line, or solid line), shadow, thickness, and color, etc.

The coordinate system of the above-mentioned position coordinates is a two-dimensional or three-dimensional coordinate system established by using a centroid of the ablation site in the displayed image or an end point of the image as the origin.

Optionally, different colors and/or different types of lines can be used respectively in drawing the target operation track and the real-time operation track, to highlight the difference between target operation track and the real-time operation track, thereby further improving the effectiveness of information prompts.

Further, when the real-time operation track is drawn, the position coordinate of each ablation point in the drawn real-time operation track is compared with the position coordinate of each corresponding ablation point in the target operation track (corresponding to the ablation point in the real-time operation track in the drawing order), to obtain the coordinate variation therebetween. Then, the number of ablation points with a coordinate variation that is greater than a preset variation is counted. If the number is greater than a preset number, the amplitude of the real-time operation track deviating from the target operation track is determined to be greater than a preset amplitude, and prompt information for track deviation warning is outputted. Optionally, the prompt information can be in the form of a text and/or a graphic, and displayed in the first preset area of the prompting interaction interface.

Therefore, the target operation track and the real-time operation track are drawn, and the amplitude of deviation therebetween is analyzed. When the amplitude of deviation between the two is greater than the preset amplitude, the prompt information is displayed, so as to avoid the adverse effect of the user's improper operation on the ablation effect, improve the universality and intelligence of information prompts, and further improve the effectiveness of information prompts.

Optionally, in another embodiment of the present application, the method further includes:

acquiring the temperature of the ablation site in real time when the ablation task is triggered;
drawing a real-time temperature change curve according to the temperature acquired in real time and displaying it on the screen; and
analyzing in real time whether the temperature exceeds a preset warning value, and outputting prompt information for temperature warning in a display area of the temperature change curve when the temperature exceeds the warning value.

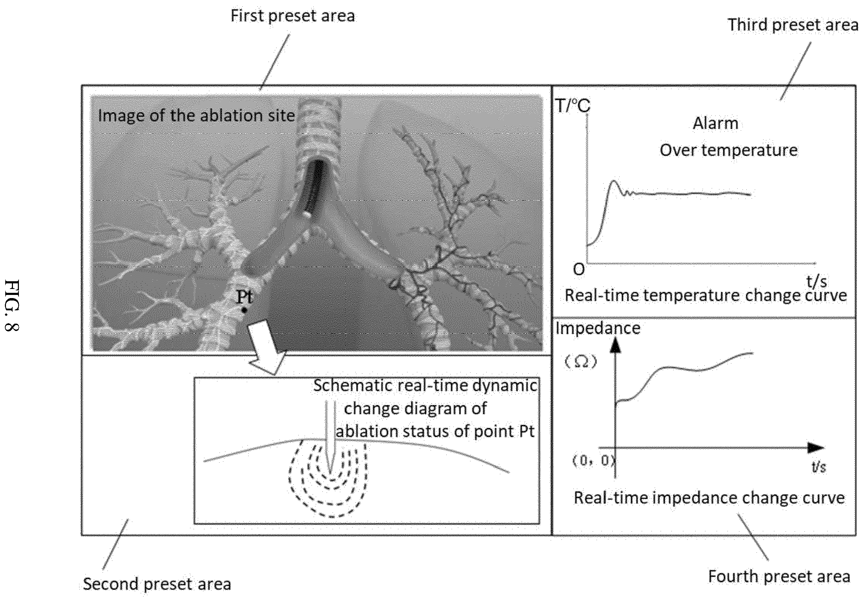

Specifically, the temperature of the ablation site can be obtained in real time by a temperature sensor set near the ablation site, and then a real-time temperature change curve as shown in FIG. 8 is drawn by calling a curve drawing function (such as: PLOT function) according to the temperature acquired in real time. The horizontal axis of the real-time temperature change curve is a time axis, and used to indicate the time at which each temperature value is obtained (t/s). The vertical axis of the real-time temperature change curve represents the temperature (T/°C). Optionally, the real-time temperature change curve can be drawn in a third preset area of the prompting interaction interface.

It can be understood that in addition to the PLOT function, many more functions are available for curve drawing, and the function used can be selected according to actual needs in practical applications and is not particularly limited in the present application.

Further, when the real-time temperature change curve is drawn, whether the obtained temperature exceeds a preset warning value is analyzed in real time, and outputting prompt information for temperature warning in a display area (for example, the third preset area) of the temperature change curve when the temperature exceeds the warning value. The prompt information for temperature warning can be displayed in the form of a text and/or a graphic, as shown FIG. 8.

Therefore, by drawing the real-time temperature change curve, and when the acquired temperature exceeds the warning value, warning information is outputted. This promotes the user to understand the temperature change of the ablation site in time and achieve the effect of temperature warning, thus further improving the universality and effectiveness of information prompts, and improving the safety of the ablation operation. In addition, by outputting the prompt information for temperature warning in the display area of the temperature change curve, the prompt information is made more directional.

Optionally, in another embodiment of the present application, the method further includes:

acquiring the impedance of the ablation site in real time when the ablation task is triggered; and
drawing a real-time impedance change curve on a screen according to the impedance obtained in real time.

Specifically, by setting an impedance sensor installed at a tip of the radio-frequency ablation catheter, the impedance of the ablation site is acquired in real time; and a real-time impedance change curve as shown in FIG. 8 is drawn on the screen, by calling the above curve drawing function, according to the acquired impedance. The horizontal axis of the real-time impedance change curve represents time (t/s). The vertical axis of the real-time impedance change curve represents the impedance (Q). Optionally, the real-time impedance change curve can be drawn in a fourth preset area of the prompting interaction interface.

Therefore, by drawing the real-time impedance change curve, the user is promoted to understand the impedance changes of the ablation site in time, to adjust the ablation operation in time, thus further improving the universality and effectiveness of information prompts, and improving the safety of the ablation operation.

Optionally, in another embodiment of the present application, the method further includes:

acquiring the impedance of the ablation site in real time, when the ablation task is triggered;
determining a target interval corresponding to the impedance according to the impedance acquired in real time and the impedance ranges respectively corresponding to multiple preset ablation impedance prompting intervals; and
drawing a schematic real-time impedance diagram on the screen according to the impedance, the impedance range and the target interval. The schematic real-time impedance diagram includes: description information of the multiple ablation impedance prompting intervals, description information of a preset reference impedance, and description information of the corresponding relationship between the impedance and the target interval. The multiple ablation impedance prompting intervals include: a non-ablatable impedance interval, an ablatable impedance interval, and an optimal ablatable impedance interval.

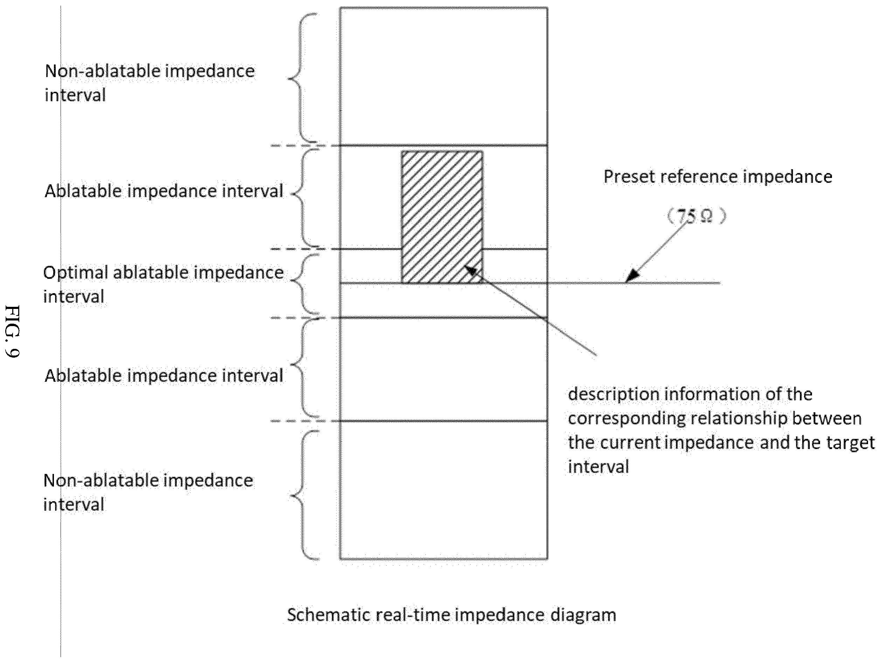

Further, as shown in FIG. 9, the description information of the multiple ablation impedance prompting intervals includes: multiple columns stacked one above another vertically (six columns in FIG. 9, and the number is not limited to six in actual applications) and the corresponding description texts and indicating graphics (such as the dashed line and curly brackets in FIG. 9) of the multiple ablation impedance prompting intervals; and the description information of the preset reference impedance includes: the description text and the indicating graphic (for example, arrow) of the preset reference impedance.

The multiple columns respectively correspond, from top to bottom, to an upper range of the non-ablatable impedance interval, an upper range of the ablatable impedance interval, an upper range of the optimal ablatable impedance interval, a lower range of the optimal ablatable impedance interval, a lower range of the ablatable impedance interval, and a lower range of the non-ablatable impedance interval.

The upper and lower limits of the non-ablatable impedance interval, ablatable impedance interval, and the optimal ablatable impedance interval can be preset in the radio-frequency ablation control device according to the user's custom operation. Optionally, the preset reference impedance may be a median value of the optimal ablation impedance interval or an average value of the upper limit and the lower limit. In this case, if the preset reference impedance is set according to the user's custom operation, the upper and lower limits of the non-ablatable impedance interval, the ablatable impedance interval, and optimal ablation impedance interval can be determined according to the preset reference impedance and the fluctuation range of each impedance interval preset by the user.

Further, the number of columns, the number and range of the ablation impedance prompting interval, and the correspondence between the column and each ablation impedance prompting interval may also be determined according to the number of electrodes on the radio-frequency ablation catheter, or set according to the user's custom operation.

Further, taking the column corresponding to the optimal ablatable impedance interval as a center, the height increases as the distance of the other columns in the multiple columns from the column increases. Therefore, by setting different sizes of columns to identify different ablation impedance intervals, the user is allowed to get to know the ablation impedance interval corresponding to the current impedance clearly, thereby further improving the eye-catching and intuitiveness of the prompt information, and improving the effectiveness of information prompts.

Optionally, in another embodiment of the present application, the method further includes:

acquiring the impedance of the ablation site in real time when the ablation task is triggered;

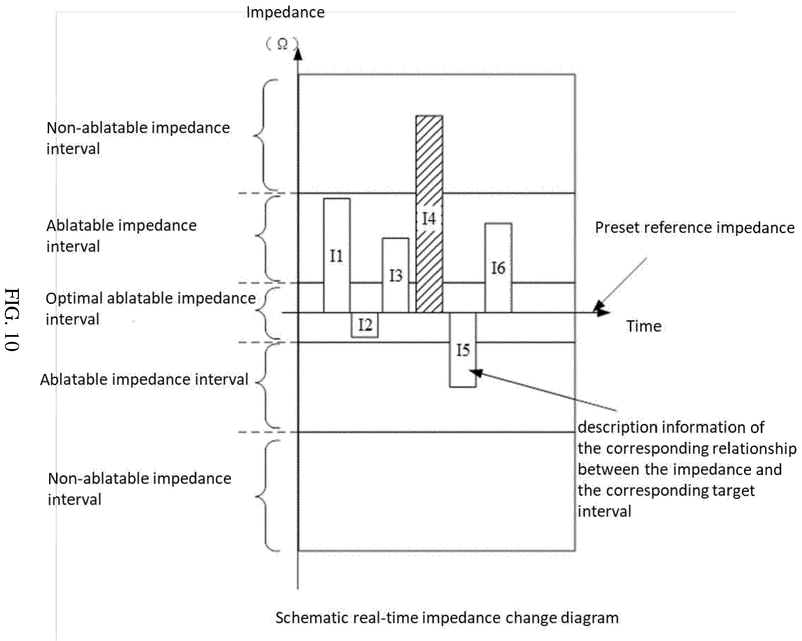

determining a target interval corresponding to the impedance according to the impedance acquired in real time and the impedance ranges respectively corresponding to multiple preset ablation impedance prompting intervals; and

drawing a schematic real-time impedance change diagram on the screen (as shown in FIG. 10) according to the impedance, the impedance range and the target interval. The schematic real-time impedance change diagram includes: two-dimensional coordinate axes, description information of the multiple ablation impedance prompting intervals, and description information of the corresponding relationship between each impedance and respective target interval. The multiple ablation impedance prompting intervals include: a non-ablatable impedance interval, an ablatable impedance interval, and an optimal ablatable impedance interval.

The horizontal axis of the two-dimensional coordinate axes is a time axis, indicating the acquisition time of each impedance. Moreover, the horizontal axis is also used to indicate the preset reference impedance. The vertical axis indicates, from bottom to top in a positive direction, an upper range of the optimal ablatable impedance interval, an upper range of the ablatable impedance interval, and an upper range of the non-ablatable impedance interval, and indicates, from top to bottom in a negative direction, a lower range of the optimal ablatable impedance interval, a lower range of the ablatable impedance interval, and a lower range of the non-ablatable impedance interval.

Optionally, the schematic real-time impedance change diagram is drawn on the preset prompting interaction interface.

Therefore, by drawing the schematic real-time impedance change diagram, the user is promoted to get to know the impedance changes of the ablation site in real time, and the user is reminded to adjust the ablation operation in time by an impedance warning, thus further improving the universality and effectiveness of information prompts.

Optionally, in another embodiment of the present application, the description information of the corresponding relationship between the impedance and the target interval includes: graphics of different colors with preset shapes, wherein the different colors respectively correspond to different ablation impedance prompting intervals. For example, the red color corresponds to the non-ablatable impedance interval, the yellow color corresponds to the ablatable impedance interval, and the green color corresponds to the optimal ablatable impedance interval. Therefore, by using different colors, different ablation impedance intervals can be effectively distinguished, thereby further improving the effectiveness of information prompts.

Optionally, the height of the graphic is determined according to the difference between the impedance and the upper limit or lower limit of the corresponding target interval.

Preferably as shown in FIG. 10, the graphic has a bar shape, and as the impedance approaches the limit of the corresponding target interval, the length of the bar increases. The schematic real-time impedance change diagram can not only indicate the user with the real-time impedance change, but also indicate the user whether there is a safety risk in the current ablation operation. For example, the target interval corresponding to the impedance 12 in FIG. 10 is an optimal ablatable impedance interval, and indicating that the ablation operation at this time can achieve the best ablation effect; and the target interval corresponding to the impedance 14 is a non-ablatable impedance interval, indicating that the impedance of the ablation site is too high or too low, and there is a safety risk in the current ablation operation.

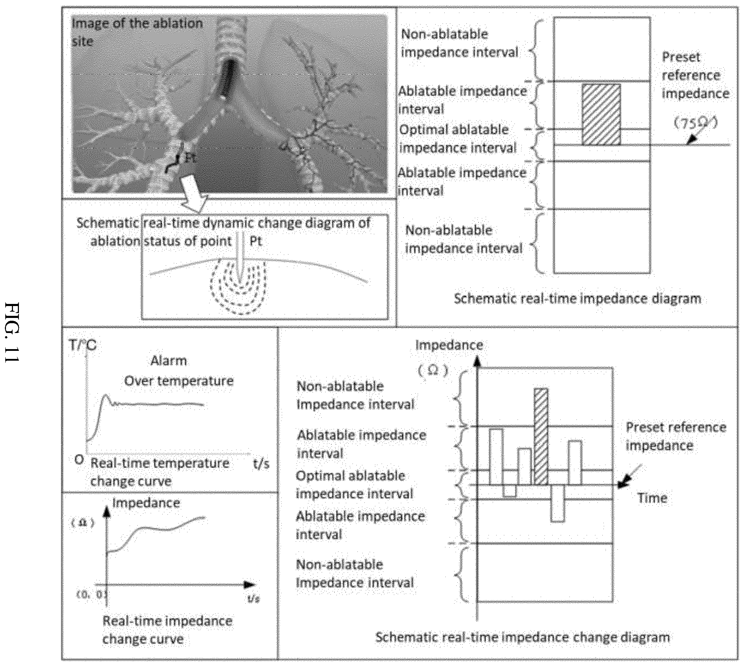

It should be noted that the schematic real-time impedance diagram, the schematic real-time impedance change diagram, the real-time impedance change curve, and other schematic diagrams do not conflict with each other. In practical use, one or more schematic diagrams illustrating a selective operation can be drawn and displayed according to the selective operation of the user. For example, as shown in FIG. 11, all the schematic diagrams are displayed on the preset prompting interaction interface. FIG. 4, FIG. 5, and FIGs. 7 to 11 are merely exemplary. In practical use, other more or less information, for example, specific temperature, impedance, system time, described information of the ablation object, described information of the person in charge of the ablation operation, can be included in a different arrangement according to the practical needs or user's choice.

Further, the radio-frequency ablation catheter includes a single-electrode radio-frequency ablation catheter and a multi-electrode radio-frequency ablation catheter, wherein the single-electrode radio-frequency ablation catheter is correspondingly provided with a single impedance sensor, and the multi-electrode radio-frequency ablation catheter is correspondingly provided with multiple impedance sensors. The target interval can be determined according to the type of radio-frequency ablation catheter. Specifically, the determining a target interval corresponding to the impedance, according to the impedance acquired in real time and the impedance ranges respectively corresponding to multiple preset ablation impedance prompting intervals, includes:

when the radio-frequency ablation catheter is a single-electrode radio-frequency ablation catheter, determining a target interval corresponding to the single impedance in real time according to the single impedance acquired in real time and the impedance ranges respectively corresponding to multiple ablation impedance prompting intervals; and

when the radio-frequency ablation catheter is a multi-electrode radio-frequency ablation catheter, analyzing whether the multiple impedances obtained in real time correspond to the same ablation impedance prompting interval according to the impedance ranges respectively corresponding to multiple ablation impedance prompting intervals, wherein

if the multiple impedances correspond to the same ablation impedance prompting interval, the corresponding ablation impedance prompting interval is used as the target interval, and

if the multiple impedances correspond to multiple ablation impedance prompting intervals, an ablation impedance prompting interval that covers most of the multiple impedances is used as the target interval.

Specifically, before implementing an ablation task, the radio-frequency ablation control device acquires model information of the connected radio-frequency ablation catheter, determines the type of the radio-frequency ablation catheter according to the acquired model information. Then, the target interval is determined according to the type determined. Taking a six-electrode radio-frequency ablation catheter as an example, the six-electrode radio-frequency ablation catheter is assumed to be correspondingly provided with 6 impedance sensors, if the 6 impedances obtained by the 6 impedance sensors all fall in the optimal ablatable impedance interval, the corresponding target interval is determined to be the optimal ablatable impedance interval, and if 4 of the 6 impedances fall in the ablatable impedance interval and 2 impedances fall in the optimal ablatable impedance interval, the corresponding target interval is determined to be the ablatable impedance interval.

Optionally, in another embodiment of the present application, after Step S601, the method further includes:

when the prompt information outputted on the screen or the drawn schematic diagram has target information containing preset keywords, performing a screencapture operation, and saving the captured picture; and

displaying all the pictures saved on the screen according to the priority of the target information, after the ablation task is ended.

Specifically, whether the prompt information outputted on the screen or the drawn schematic diagram has target information containing preset keywords is analyzed in real time, wherein the preset keywords have alert meaning, and may include, but is not limited to, for example: alarm, reminder, attention, and warning. If there is target information that contains the preset keywords, a screencapture operation is performed, and the captured picture is stored in the radio-frequency ablation control device locally or at a preset location of a cloud server. Then, after the ablation task is ended, all the pictures saved are displayed on the screen according to the timing sequence upon capture or the priority of the target information, to indicate to the user the abnormal conditions during the implementation of the entire ablation operation, thereby further improving the scope of application of information prompts, and improve the universality and intelligence of information prompts. A higher priority of the target information indicates a higher severity or importance of the corresponding prompt information.

Optionally, when the preset prompting interaction interface is used as a carrier, whether the prompt information outputted on the prompting interaction interface or the drawn schematic diagram has target information containing preset keywords is analyzed in real time.

It should be noted that for ease of description, the steps in each embodiment of the present application are numbered in sequence; however, the numbering sequence does not constitute a restriction on the order of implementation. Some steps can be implemented at the same time, for example, Step S602 and Step S603 can be implemented at the same time, Step S604 and step S605 can also be implemented at the same time, and the generation and display of other schematic diagrams involved may also be implemented at the same time.

In the embodiments of this application, when an ablation task is triggered, an image of an ablation site is acquired and displayed on a preset prompting interaction interface where the image of the ablation site is marked with a currently-being-ablated target ablation point; according to the elapsed ablation time and the temperature of the target ablation point acquired in real time, a schematic image showing the real-time dynamic change of the ablation status of the target ablation point is generated and displayed, so that the status changes of an ablation site can be displayed in real time and intuitively during the implementation of the ablation operation, thereby improving the effectiveness and relevance of information prompts.

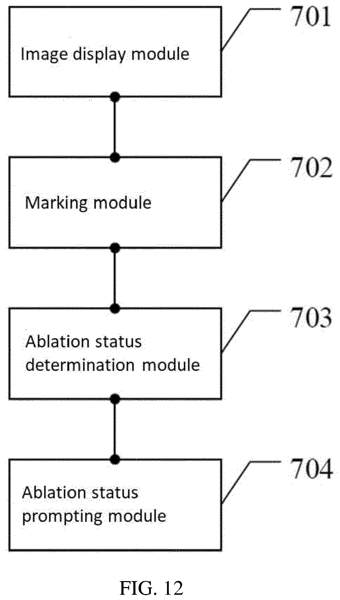

FIG. 12 is a schematic structural diagram of an ablation operation prompting device provided in an embodiment of the present application. For ease of description, only the parts relevant to the embodiments of the application are shown. The device may be a computer terminal, or a software module configured on the computer terminal. As shown in FIG. 12, the device includes an image display module 701, a marking module 702, an ablation status determination module 703 and an ablation status prompting module 704.

The image display module 701 is configured to acquire an image of an ablation site and display the image on a screen, when an ablation task is triggered.

The marking module 702is configured to acquire position data of a currently-being-ablated target ablation point, and mark the target ablation point in the image according to the position data.

The ablation status determination module 703 is configured to acquire the elapsed ablation time and the temperature of the target ablation point in real time, and determine the ablation status of the target ablation point according to the elapsed ablation time and the temperature.

The ablation status prompting module 704 is configured to generate a schematic diagram showing the real-time dynamic change of the target ablation point according to the ablation status, and display the schematic diagram on the screen, to indicate the real-time ablation status change of the target ablation point.

Optionally, the device further includes:

a target operation track drawing module, configured to acquire a first drawing parameter of a target operation track of the ablation operation, and draw the target operation track at a corresponding position of the image according to the first drawing parameter;

a real-time operation track drawing module, configured to acquire the position change data of the ablation point in real time, determine a second drawing parameter of a real-time operation track of the ablation operation according to the position change data, and draw the real-time operation track at a corresponding position of the image according to the second drawing parameter; and

a track warning module, configured to analyze in real time whether the amplitude of the real-time operation track deviating from the target operation track is greater than a preset amplitude, and outputting prompt information for track deviation warning when the real-time operation track deviates from the target operation track by an amplitude greater than the preset amplitude.

Optionally, the device further includes:

a temperature warning module, configured to acquire the temperature of the ablation site in real time, when the ablation task is triggered; draw a real-time temperature change curve on the screen according to the temperature acquired in real time; and analyze in real time whether the temperature exceeds a preset warning value, and outputting prompt information for temperature warning in a display area of the temperature change curve when the temperature exceeds the warning value.

Optionally, the device further includes:

an impedance acquisition module, configured to acquire the impedance of the ablation site in real time, when the ablation task is triggered; and

a real-time impedance change curve drawing module, configured to draw a real-time impedance change curve on the screen according to the impedance obtained in real time.

Optionally, the device further includes:

a target interval determination module, configured to determine a target interval corresponding to the impedance, according to the impedance acquired in real time and the impedance ranges respectively corresponding to multiple preset ablation impedance prompting intervals; and

a schematic real-time impedance diagram drawing module, configured to draw a schematic real-time impedance diagram on the screen according to the impedance, the impedance range and the target interval. The schematic real-time impedance diagram includes: description information of the multiple ablation impedance prompting intervals, description information of a preset reference impedance, and description information of the corresponding relationship between the impedance and the target interval. The multiple ablation impedance prompting intervals include: a non-ablatable impedance interval, an ablatable impedance interval, and an optimal ablatable impedance interval.

Optionally, the description information of the multiple ablation impedance prompting intervals includes: multiple columns stacked one above another vertically and the corresponding description texts and indicating graphics of the multiple ablation impedance prompting intervals. The description information of the preset reference impedance includes: the description text and the indicating graphic of the preset reference impedance.

The multiple columns are respectively corresponding to, from top to bottom, an upper range of the non-ablatable impedance interval, an upper range of the ablatable impedance interval, an upper range of the optimal ablatable impedance interval, a lower range of the optimal ablatable impedance interval, a lower range of the ablatable impedance interval, and a lower range of the non-ablatable impedance interval.

Taking the column corresponding to the optimal ablatable impedance interval as a center, the height of the other column of the multiple columns increases as the distance of the other column from the column increases.

Optionally, the device further includes:

a schematic real-time impedance change diagram drawing module, configured to draw a schematic real-time impedance change diagram on the screen according to the impedance, the impedance range and the target interval. The schematic real-time impedance change diagram includes: two-dimensional coordinate axes, description information of the multiple ablation impedance prompting intervals, and description information of the corresponding relationship between each impedance and respective target interval. The multiple ablation impedance prompting intervals include: a non-ablatable impedance interval, an ablatable impedance interval, and an optimal ablatable impedance interval.

The horizontal axis of the two-dimensional coordinate axes is a time axis, indicating the acquisition time of each impedance and also for indicating the preset reference impedance. The vertical axis of the two-dimensional coordinate axes indicates, from bottom to top in a positive direction, an upper range of the optimal ablatable impedance interval, an upper range of the ablatable impedance interval, and an upper range of the non-ablatable impedance interval, and indicates, from top to bottom in a negative direction, a lower range of the optimal ablatable impedance interval, a lower range of the ablatable impedance interval, and a lower range of the non-ablatable impedance interval.

Optionally, the description information of the corresponding relationship includes: graphics of different colors with preset shapes, wherein the different colors respectively correspond to different ablation impedance prompting intervals.

The height of the graphic is determined according to the difference between the impedance and the upper limit or lower limit of the corresponding target interval.

Optionally, the target interval determination module is specifically configured to determine a target interval corresponding to the single impedance in real time according to the single impedance acquired in real time and the impedance ranges respectively corresponding to multiple ablation impedance prompting intervals when the radio-frequency ablation catheter is a single-electrode radio-frequency ablation catheter; and analyze whether the multiple impedances obtained in real time correspond to the same ablation impedance prompting interval according to the impedance ranges respectively corresponding to multiple ablation impedance prompting intervals, when the radio-frequency ablation catheter is a multi-electrode radio-frequency ablation catheter, determine the corresponding ablation impedance prompting interval as the target interval ,if the multiple impedances correspond to the same ablation impedance prompting interval, and an ablation impedance prompting interval that covers the most impedances is used as the target interval.

Optionally, the marking module 702 includes:

a first positioning module, configured to acquire a picture of a current ablation operation captured by an endoscope and compare the captured picture with the image, to obtain the position data of the target ablation point in the image.

Optionally, the marking module 702 further includes:

a second positioning module, configured to obtain an ultrasound image of the target ablation point; and acquire the position data of the target ablation point in the image according to the ultrasound image.

Optionally, the marking module 702 further includes:

a third positioning module, configured to acquire the position data of the target ablation point, by electromagnetic navigation technology.

Optionally, the device further includes:

a screencapture module, configured to perform a screencapture operation when the prompt information outputted on the screen or the drawn schematic diagram has target information containing preset keywords, and save the captured picture; and
a display module, configured to display all the pictures saved on the screen according to the priority of the target information, after the ablation task is ended.

Optionally, the device further includes:

an ablation status analyzing module, configured to determine whether the ablation status of the target ablation point reaches a preset target ablation status according to the elapsed ablation time and the temperature, and output prompt information indicating reaching of the target ablation status if the ablation status of the target ablation point reaches the target ablation status.

The marking module 702 is further configured to take an ablation point corresponding to a changed position as the target ablation point, when the position of the ablation operation is detected to be changed; update the mark in the image and return to implement the step of acquiring the elapsed ablation time and the temperature of the target ablation point in real time, and determining the ablation status of the target ablation point according to the elapsed ablation time and the temperature, until the ablation operation is ended.

The specific process for the above modules to implement their respective functions can be made reference to the relevant description in the embodiments shown in FIG. 3 to FIG. 11, and will not be repeated here.

In the embodiments of this application, when an ablation task is triggered, an image of an ablation site is acquired and displayed on a preset prompting interaction interface where the image of the ablation site is marked with a currently-being-ablated target ablation point; according to the elapsed ablation time and the temperature of the target ablation point acquired in real time, a schematic image showing the real-time dynamic change of the ablation status of the target ablation point is generated and displayed, so that the status changes of an ablation site can be displayed in real time and intuitively during the implementation of the ablation operation, thereby improving the effectiveness and relevance of information prompts.

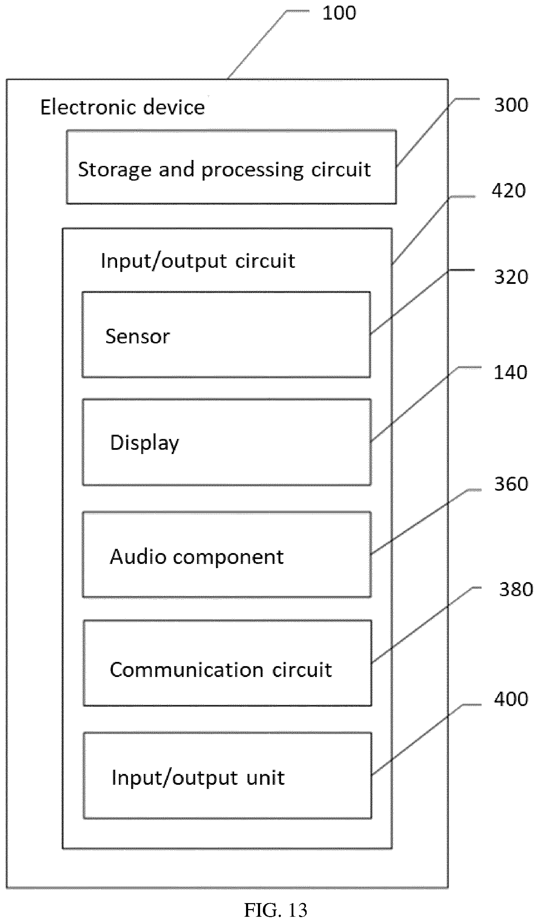

FIG. 13 is a schematic diagram showing a hardware structure of an electronic device provided in an embodiment of the present application.

Exemplarily, the electronic device may be any of various types of computer devices that are non-movable or movable or portable and capable of wireless or wired communication. Specifically, the electronic device can be a desktop computer, a server, a mobile phone or smart phone (e.g., a phone based on iPhone TM, and Android TM), a portable game device (e.g. Nintendo DS TM, PlayStation Portable TM, Gameboy Advance TM, iPhone TM), a laptop computer, PDA, a portable Internet device, a portable medical device, a smart camera, a music player, a data storage device, and other handheld devices such as watches, earphones, pendants, and headphones, etc. The electronic device may also be other wearable devices (for example, electronic glasses, electronic clothes, electronic bracelets, electronic necklaces and other head-mounted devices (HMD)).

In some cases, the electronic device can implement a variety of functions, for example: playing music, displaying videos, storing pictures and receiving and sending phone calls.

As shown in FIG. 13, the electronic device 100 includes a control circuit, and the control circuit includes a storage and processing circuit 300. The storage and processing circuit 300 includes a storage, for example, hard drive storage, non-volatile storage (such as flash memory or other storages that are used to form solid-state drives and are electronically programmable to confine the deletion, etc.), and volatile storage (such as static or dynamic random access storage) which is not limited in the embodiments of the present application. The processing circuit in the storage and processing circuit 300 can be used to control the operation of the electronic device 100. The processing circuit can be implemented based on one or more microprocessors, microcontrollers, digital signal processors, baseband processors, power management units, audio codec chips, application specific integrated circuits, and display driver integrated circuits.

The storage and processing circuit 300 can be used to run the software in the electronic device 100, such as Internet browsing applications, Voice over Internet Protocol (VOIP) phone call applications, Email applications, media player applications, and functions of operating system. The software can be used to perform some control operations, for example, camera-based image acquisition, ambient light measurement based on an ambient light sensor, proximity sensor measurement based on a proximity sensor, information display function implemented by a status indicator based on a status indicator lamp such as light emitting diode, touch event detection based on a touch sensor, functions associated with displaying information on multiple (e.g. layered) displays, operations associated with performing wireless communication functions, operations associated with the collection and generation of audio signals, control operations associated with the collection and processing of button press event data, and other functions in electronic device 100, which are not limited in the embodiments of the present application.

Further, the storage stores an executable program code. The processor coupled to the storage calls the executable program code stored in the storage, and implement the ablation operation prompting method described in the embodiments shown in FIGs. 3 to 11.

The executable program code includes various modules in the ablation operation prompting device described in the embodiment shown in FIG. 12, for example, the image display module 701, the marking module 702, the ablation status determination module 703, and the ablation status prompting module 704. The specific process for the above modules to implement their functions can be made reference to the relevant description in the embodiments shown in FIG. 12, and will not be repeated here.

The electronic device 100 may also include an input/output circuit 420. The input/output circuit 420 can be used to enable the electronic device 100 to implement the input and output of data, that is, the electronic device 100 is allowed to receive data from an external device and the electronic device 100 is also allowed to output data from the electronic device 100 to the external device. The input/output circuit 420 may further include a sensor 320. The sensor 320 may include one or a combination of an ambient light sensor, a proximity sensor based on light and capacitance, a touch sensor (e.g., light-based touch sensor and/or capacitive touch sensor, wherein the touch sensor may be part of the touch screen, or it can also be used independently as a touch sensor structure), an accelerometer, and other sensors, etc.

The input/output circuit 420 may also include one or more displays, for example, display 140. The display 140 may include a liquid crystal display, an organic light emitting diode display, an electronic ink display, a plasma display, and a display that uses other display technologies. The display 140 may include a touch sensor array (i.e., the display 140 may be a touch display screen). The touch sensor can be a capacitive touch sensor formed by an array of transparent touch sensor electrodes (such as indium tin oxide (ITO) electrodes), or a touch sensor formed using other touch technologies, for example, sonic touch, pressure sensitive touch, resistive touch, and optical touch, which is not limited in the embodiments of the present application.

The electronic device 100 can also include an audio component 360. The audio component 360 can be used to provide audio input and output functions for the electronic device 100. The audio component 360 in the electronic device 100 includes a speaker, a microphone, a buzzer, and a tone generator and other components used to generate and detect sound.

A communication circuit 380 can be used to provide the electronic device 100 with an ability to communicate with an external device. The communication circuit 380 may include an analog and digital input/output interface circuit, and a wireless communication circuit based on radio-frequency signals and/or optical signals. The wireless communication circuit in the communication circuit 380 may include a radio-frequency transceiver circuit, a power amplifier circuit, a low-noise amplifier, a switch, a filter, and an antenna. For example, the wireless communication circuit in the communication circuit 380 may include a circuit for supporting near field communication (NFC) by transmitting and receiving near-field coupled electromagnetic signals. For example, the communication circuit 380 may include a near-field communication antenna and a near-field communication transceiver. The communication circuit 380 may also include a cellular phone transceiver and antenna, and a wireless LAN transceiver circuit and antenna, etc.

The electronic device 100 may also further include a battery, a power management circuit and other input/output units 400. The input/output unit 400 may include a button, a joystick, a click wheel, a scroll wheel, a touchpad, a keypad, a keyboard, a camera, a light-emitting diode and other status indicators, etc.

The user can control the operation of the electronic device 100 by inputting a command through the input/output circuit 420, and the output data from the input/output circuit 420 enables the receiving of status information and other outputs from the electronic device 100.

Further, an embodiment of the present application further provides a non-transitory computer-readable storage medium. The non-transitory computer-readable storage medium can be configured in the server in each of the above embodiments, and a computer program is stored in the non-transitory computer-readable storage medium. When the program is executed by the processor, the ablation operation prompting method described in the embodiments shown in FIG. 3 to FIG. 11 is implemented.

In the embodiments described above, emphasis has been placed on the description of various embodiments. Parts of an embodiment that are not described or illustrated in detail may be found in the description of other embodiments.

Those of ordinary skill in the art will recognize that the exemplary modules/units and algorithm steps described in connection with the embodiments disclosed herein may be implemented by electronic hardware, or a combination of computer software and electronic hardware. Whether such functions are implemented by hardware or software depends on the particular application and design constraints of the technical solutions. Skilled artisans may implement the described functions in varying ways for each particular application. but such implementation is not intended to exceed the scope of the present invention.

In the embodiments provided in the present invention, it can be understood that the disclosed device/terminal and method may be implemented in other ways. For example, the device/terminal embodiments described above are merely illustrative. For example, a division of the modules or elements is merely division of logical functions, and there may be additional divisions in actual implementation. For example, multiple units or components may be combined or integrated into another system, or some features may be omitted or not performed. Alternatively, the couplings or direct couplings or communicative connections shown or discussed with respect to one another may be indirect couplings or communicative connections via some interfaces, devices or units may be electrical, mechanical or otherwise.

The units described as separate components may or may not be physically separate, and the components shown as units may or may not be physical units, i.e. may be located in one place, or may be distributed over a plurality of network elements. Some or all of the units may be selected to achieve the objectives of the solution of the present embodiment according to practical requirements.

In addition, the functional units in the various embodiments of the present invention may be integrated into one processing unit, may be physically separate from each other or may be integrated in one unit by two or more units. The integrated units described above can be implemented either in the form of hardware, or software functional units.

The integrated unit, if implemented in the form of a software functional unit and sold or used as a stand-alone product, may be stored in a computer readable storage medium. Based on such an understanding, all or part of the processes of the methods in the above-described embodiments implemented in the present application, may also be implemented by a computer program instructing related hardware. The computer program may be stored in a computer-readable storage medium, and performs the steps of the various method embodiments described above when executed by the processor. The computer program includes a computer program code, which may be in the form of source code, object code, or executable file, or in some intermediate form. The computer readable medium may include: any entity or device capable of carrying the computer program code, recording media, U disks, removable hard disks, magnetic disks, optical disks, computer memory, read-only memory (ROM), random access memory (RAM), electric carrier wave signals, telecommunication signals and software distribution media. It should be noted that the computer readable medium may contain content that may be appropriately augmented or subtracted as required by legislation and patent practice within judicial jurisdictions, e.g., the computer-readable medium does not include electrical carrier wave signals and telecommunications signals in accordance with legislation and patent practices in some jurisdictions.

The above-described embodiments are merely illustrative of, and not intended to limit the technical solutions of the present invention. Although the present invention has been described in detail with reference to the foregoing embodiments, it should be understood by those of ordinary skill in the art that the technical solutions of the above-mentioned embodiments can still be modified, or some of the technical features thereof can be equivalently substituted; and such modifications and substitutions do not cause the nature of the corresponding technical solution to depart from the spirit and scope of the embodiments of the present disclosure and are intended to be included within the scope of this application.

**Claims**

1. An ablation operation prompting method, applicable to a computer terminal, comprising steps of:

   acquiring an image of an ablation site and displaying the image on a screen when an ablation task is triggered;
   acquiring position data of a currently-being-ablated target ablation point, and marking the target ablation point in the image according to the position data;
   acquiring elapsed ablation time and temperature of the target ablation point in real time, and determining the ablation status of the target ablation point according to the elapsed ablation time and the temperature; and
   generating a schematic real-time dynamic change diagram of the target ablation point according to the ablation status, and displaying the schematic diagram on the screen, to indicate a real-time ablation status change of the target ablation point.

2. The method according to claim 1, wherein after the step of acquiring an image of an ablation site and displaying the image on a screen when an ablation task is triggered, the method further comprises steps of:

   acquiring a first drawing parameter of a target operation track of the ablation operation, and drawing the target operation track at a corresponding position of the image according to the first drawing parameter;
   acquiring position change data of the ablation point in real time, determining a second drawing parameter of a real-time operation track of the ablation operation according to the position change data, and drawing the real-time operation track at a corresponding position of the image according to the second drawing parameter; and
   analyzing in real time whether the amplitude of the real-time operation track deviating from the target operation track is greater than a preset amplitude, and outputting prompt information for track deviation warning when the real-time operation track deviates from the target operation track by an amplitude greater than the preset amplitude.

3. The method according to claim 1, wherein the method further comprises steps of:

   acquiring temperature of the ablation site in real time when the ablation task is triggered;
   drawing a real-time temperature change curve on the screen according to the temperature acquired in real time; and
   analyzing in real time whether the temperature exceeds a preset warning value, and outputting prompt information for temperature warning in a display area of the temperature change curve when the temperature exceeds the warning value.

4. The method according to claim 1, wherein the method further comprises steps of:

   acquiring impedance of the ablation site in real time when the ablation task is triggered; and
   drawing a real-time impedance change curve on the screen according to the impedance obtained in real time.

5. The method according to claim 1, wherein the method further comprises steps of:

   acquiring impedance of the ablation site in real time when the ablation task is triggered;
   determining a target interval corresponding to the impedance according to the impedance acquired in real time and impedance ranges respectively corresponding to multiple preset ablation impedance prompting intervals; and

drawing a schematic real-time impedance diagram on the screen according to the impedance, the impedance range and the target interval, wherein the schematic real-time impedance diagram comprises: description information of the multiple ablation impedance prompting intervals, description information of a preset reference impedance, and description information of the corresponding relationship between the impedance and the target interval; and the multiple ablation impedance prompting intervals comprise: a non-ablatable impedance interval, an ablatable impedance interval, and an optimal ablatable impedance interval.

6. The method according to claim 5, wherein the description information of the multiple ablation impedance prompting intervals comprises: multiple columns stacked one above another vertically and corresponding description texts and indicating graphics of the multiple ablation impedance prompting intervals, and the description information of the preset reference impedance comprises: description text and a indicating graphic of the preset reference impedance,

wherein the multiple columns are respectively corresponding to, from top to bottom, an upper range of the non-ablatable impedance interval, an upper range of the ablatable impedance interval, an upper range of the optimal ablatable impedance interval, a lower range of the optimal ablatable impedance interval, a lower range of the ablatable impedance interval, and a lower range of the non-ablatable impedance interval;

taking the column corresponding to the optimal ablatable impedance interval as a center, a height of an other column of the multiple columns increases as a distance of the other column from the column increases.

7. The method according to claim 1, wherein the method further comprises steps of:

acquiring impedance of the ablation site in real time when the ablation task is triggered;

determining a target interval corresponding to the impedance according to the impedance acquired in real time and impedance ranges respectively corresponding to multiple preset ablation impedance prompting intervals; and

drawing a schematic real-time impedance change diagram on the screen according to the impedance, the impedance range and the target interval, wherein the schematic real-time impedance change diagram comprises: two-dimensional coordinate axes, and description information of the multiple ablation impedance prompting intervals, and description information of the corresponding relationship between each impedance and respective target interval; and the multiple ablation impedance prompting intervals comprise: a non-ablatable impedance interval, an ablatable impedance interval, and an optimal ablatable impedance interval,

wherein a horizontal axis of the two-dimensional coordinate axes is a time axis, indicating the acquisition time of each impedance and also for indicating the preset reference impedance; and a vertical axis of the two-dimensional coordinate axes indicates, from bottom to top in a positive direction, an upper range of the optimal ablatable impedance interval, an upper range of the ablatable impedance interval, and an upper range of the non-ablatable impedance interval, and indicates, from top to bottom in a negative direction, a lower range of the optimal ablatable impedance interval, a lower range of the ablatable impedance interval, and a lower range of the non-ablatable impedance interval.

8. The method according to any one of claims 5 to 7, wherein the description information of the corresponding relationship comprises: graphics of different colors with preset shapes, wherein the different colors respectively correspond to different ablation impedance prompting intervals; and

the graphic has a height, which is determined according to a difference between the impedance and the upper limit or lower limit of the corresponding target interval.

9. The method according to any one of claims 5 to 7, wherein the step of determining a target interval corresponding to the impedance according to the impedance acquired in real time and impedance ranges respectively corresponding to multiple preset ablation impedance prompting intervals comprises:

when the radio-frequency ablation catheter is a single-electrode radio-frequency ablation catheter, determining a target interval corresponding to a single impedance in real time according to the single impedance acquired in real time and the impedance ranges respectively corresponding to multiple ablation impedance prompting intervals; and

when the radio-frequency ablation catheter is a multi-electrode radio-frequency ablation catheter, analyzing whether multiple impedances acquired in real time correspond to a same ablation impedance prompting interval according to the impedance ranges respectively corresponding to multiple ablation impedance prompting intervals, wherein
when the multiple impedances correspond to the same ablation impedance prompting interval, the corresponding ablation impedance prompting interval is used as the target interval, and when the multiple impedances correspond to multiple ablation impedance prompting intervals, an ablation impedance prompting interval that covers most of the multiple impedances is used as the target interval.

10. The method according to claim 1, wherein the step of acquiring position data of a currently-being-ablated target ablation point comprises:

acquiring a picture of a current ablation operation captured by an endoscope; and
comparing the captured pictures with the image, to obtain the position data of the target ablation point in the image.

11. The method according to claim 1, wherein the step of acquiring position data of a currently-being-ablated target ablation point comprises:

acquiring an ultrasound image of the target ablation point; and
acquiring the position data of the target ablation point in the image according to the ultrasound image.

12. The method according to claim 1, wherein the step of acquiring position data of a currently-being-ablated target ablation point comprises:
acquiring the position data of the target ablation point by electromagnetic navigation technology.

13. The method according to any one of claims 1 to 7, wherein after the step of acquiring an image of an ablation site and displaying the image on a screen when an ablation task is triggered, the method further comprises:

performing a screencapture operation when the prompt information outputted on the screen or the drawn schematic diagram has target information comprising preset keywords, and saving the captured picture; and
after the ablation task is ended, displaying all the pictures saved on the screen according to a priority of the target information.

14. The method according to claim 1, wherein after the step of marking the target ablation point in the image according to the position data, the method further comprises:

determining whether the ablation status of the target ablation point reaches a preset target ablation status according to the elapsed ablation time and the temperature;
outputting prompt information indicating reaching of the target ablation status when the ablation status of the target ablation point reaches the target ablation status; and
taking an ablation point corresponding to a changed position as the target ablation point when the position of the ablation operation is detected to be changed, updating the mark in the image, returning to implement the step of acquiring elapsed ablation time and temperature of the target ablation point in real time, and determining the ablation status of the target ablation point according to the elapsed ablation time and the temperature, until the ablation operation is ended.

15. An ablation operation prompting device, comprising:

an image display module, configured to acquire an image of an ablation site and display the image on a screen when an ablation task is triggered;
a marking module, configured to acquire position data of a currently-being-ablated target ablation point, and mark the target ablation point in the image according to the position data;
an ablation status determination module, configured to acquire elapsed ablation time and temperature of the target ablation point in real time, and determine the ablation status of the target ablation point according to the elapsed ablation time and the temperature; and
an ablation status prompting module, configured to generate a schematic real-time dynamic change diagram of the target ablation point according to the ablation status, and display the schematic diagram on the screen, to indicate a real-time ablation status change of the target ablation point.

16. An electronic device, comprising:

a storage and a processor, wherein
the storage stores an executable program code; and
the processor is coupled to the storage, and configured to call the executable program code stored in the storage, and implement the ablation operation prompting method according to any one of claims 1 to 14.

17. A non-transitory computer-readable storage medium, on which a computer program is stored, wherein when the computer program is executed by a processor, the ablation operation prompting method according to any one of claims 1 to 14 is implemented.

Radio-frequency ablation task of XXX

Ablation subject: XXX

Person in charge: XXX

Date of implementation: x/x/x

Impedance: $60\,\Omega$

Temperature: $50\,^{\circ}\mathrm{C}$

Volume of perfusion: $10\mathrm{ml}$

FIG. 1

FIG. 2

acquiring an image of an ablation site and displaying the image on a screen, when an ablation task is triggered —— S301

acquiring position data of a currently-being-ablated target ablation point, and marking the target ablation point in the image according to the position data acquired —— S302

acquiring the elapsed ablation time and the temperature of the target ablation point in real time, and determining the ablation status of the target ablation point according to the elapsed ablation time and the temperature —— S303

generating a schematic real-time dynamic change diagram of the target ablation point according to the ablation status, and displaying the schematic diagram on the screen, to indicate the real-time ablation status change of the target ablation point —— S304

FIG. 3

First preset area

Image of the ablation site

Pt

Schematic real-time dynamic change diagram of ablation status of point Pt

Second preset area

FIG. 4

First preset area

Image of the ablation site

Pt

Schematic real-time dynamic change diagram of ablation status of point Pt

Second preset area

FIG. 5

acquiring an image of an ablation site and displaying the image on a screen, when an ablation task is triggered — S601

acquiring position data of a currently-being-ablated target ablation point, and marking the target ablation point in the image according to the position data — S602

acquiring the elapsed ablation time and the temperature of the target ablation point in real time, and determining the ablation status of the target ablation point according to the elapsed ablation time and the temperature — S603

generating a schematic real-time dynamic change diagram of the target ablation point according to the ablation status, and display the schematic diagram on the screen — S604

determining whether the ablation status of the target ablation point reaches a preset target ablation status according to the elapsed ablation time and the temperature — S605

outputting prompt information indicating reaching of the target ablation status, if the ablation status of the target ablation point reaches the target ablation status — S606

taking an ablation point corresponding to a changed position as the target ablation point, when the position of the ablation operation is detected to be changed; and updating the mark of the target ablation point in the image — S607

FIG. 6

Real-time operation track    Target operation track

FIG. 7

FIG. 8

Schematic real-time impedance diagram

FIG. 9

Impedance
(Ω)

Preset reference impedance

Time

description information of
the corresponding relationship
between the impedance and
the corresponding target
interval

I6

I5

I4

I3

I2

I1

Non-ablatable impedance
interval

Ablatable impedance
interval

Optimal ablatable impedance
interval

Ablatable impedance interval

Non-ablatable impedance
interval

Schematic real-time impedance change diagram

FIG. 10

FIG. 11

FIG. 12

100

Electronic device

300

Storage and processing circuit

420

Input/output circuit

320

Sensor

140

Display

360

Audio component

380

Communication circuit

400

Input/output unit

FIG. 13

## INTERNATIONAL SEARCH REPORT

| International application No. |
| --- |
| **PCT/CN2021/072955** |

**A. CLASSIFICATION OF SUBJECT MATTER**

A61B 18/12(2006.01)i; G06F 9/451(2018.01)i

According to International Patent Classification (IPC) or to both national classification and IPC

**B. FIELDS SEARCHED**

Minimum documentation searched (classification system followed by classification symbols)

A61B,G06F

Documentation searched other than minimum documentation to the extent that such documents are included in the fields searched

Electronic data base consulted during the international search (name of data base and, where practicable, search terms used)

CNPAT; CNKI; WPI; EPODOC: 消融, 温度, 状态, 进展, 时间, 显示, 提示, 消融状态, 射频, ablation, radio frequency, time, temperature, status, state, display

**C. DOCUMENTS CONSIDERED TO BE RELEVANT**

| Category* | Citation of document, with indication, where appropriate, of the relevant passages | Relevant to claim No. |
| --- | --- | --- |
| X | CN 107530059 A (COVIDIEN LP) 02 January 2018 (2018-01-02) description, paragraphs [0091]-[0131], and figures 1-8 | 1-3, 10-17 |
| Y | CN 107530059 A (COVIDIEN LP) 02 January 2018 (2018-01-02) description, paragraphs [0091]-[0131], and figures 1-8 | 4-9 |
| Y | CN 110325137 A (INNOBLATIVE DESIGNS INC. et al.) 11 October 2019 (2019-10-11) description, paragraph [0056] | 4-9 |
| A | CN 111870341 A (SHAOXING MEIAOXIN MAGNETIC MEDICAL TECHNOLOGY CO., LTD.) 03 November 2020 (2020-11-03) entire document | 1-17 |
| A | CN 105534593 A (SHENZHEN MINDRAY BIO-MEDICAL ELECTRONICS CO., LTD. et al.) 04 May 2016 (2016-05-04) entire document | 1-17 |
| A | CN 201365978 Y (XI'AN STERILIZATION DISINFECTION EQUIPMENT MANUFACTURING CO.) 23 December 2009 (2009-12-23) entire document | 1-17 |
| A | US 2015320479 A1 (CHENES L.L.C.) 12 November 2015 (2015-11-12) entire document | 1-17 |

☐ Further documents are listed in the continuation of Box C.  ☑ See patent family annex.

| | | |
| --- | --- | --- |
| \* | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
| --- | --- |
| **28 August 2021** | **28 September 2021** |

| Name and mailing address of the ISA/CN | Authorized officer |
| --- | --- |
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT
### Information on patent family members

International application No.

**PCT/CN2021/072955**

| Patent document cited in search report | | | Publication date (day/month/year) | Patent family member(s) | | | Publication date (day/month/year) |
|---|---|---|---|---|---|---|---|
| CN | 107530059 | A | 02 January 2018 | US | 2016317231 | A1 | 03 November 2016 |
| | | | | CA | 2982263 | A1 | 03 November 2016 |
| | | | | US | 2016317230 | A1 | 03 November 2016 |
| | | | | JP | 6796600 | B2 | 09 December 2020 |
| | | | | AU | 2016254139 | A1 | 26 October 2017 |
| | | | | US | 2016317225 | A1 | 03 November 2016 |
| | | | | US | 2016317224 | A1 | 03 November 2016 |
| | | | | EP | 3291736 | A1 | 14 March 2018 |
| | | | | JP | 2018524031 | A | 30 August 2018 |
| | | | | WO | 2016176549 | A1 | 03 November 2016 |
| | | | | US | 2016317229 | A1 | 03 November 2016 |
| CN | 110325137 | A | 11 October 2019 | WO | 2018156713 | A1 | 30 August 2018 |
| | | | | EP | 3585292 | A1 | 01 January 2020 |
| | | | | JP | 2020508152 | A | 19 March 2020 |
| | | | | US | 2018235686 | A1 | 23 August 2018 |
| CN | 111870341 | A | 03 November 2020 | | None | | |
| CN | 105534593 | A | 04 May 2016 | CN | 105534593 | B | 23 April 2019 |
| CN | 201365978 | Y | 23 December 2009 | | None | | |
| US | 2015320479 | A1 | 12 November 2015 | US | 10342606 | B2 | 09 July 2019 |
| | | | | US | 2015320481 | A1 | 12 November 2015 |
| | | | | US | 2015320478 | A1 | 12 November 2015 |
| | | | | US | 2015320480 | A1 | 12 November 2015 |
| | | | | US | 9717552 | B2 | 01 August 2017 |
| | | | | US | 10639098 | B2 | 05 May 2020 |
| | | | | US | 2020330153 | A1 | 22 October 2020 |
| | | | | US | 10111703 | B2 | 30 October 2018 |
| | | | | EP | 2942023 | A2 | 11 November 2015 |
| | | | | EP | 2942023 | B1 | 24 February 2021 |
| | | | | US | 9956032 | B1 | 01 May 2018 |

Form PCT/ISA/210 (patent family annex) (January 2015)